# EUROPEAN PATENT APPLICATION

(11) **EP 4 085 910 A1**
(43) Date of publication of application: **09.11.2022**
(21) Application number: 21305580.9
(22) Date of filing: 05.05.2021
(51) Int. Cl.: A61K 31/395, A61K 31/4709, A61K 45/06, A61P 11/00, A61P 31/16, A61K 31/4353, A61K 31/4427, A61K 31/192, A61K 31/404, A61K 31/573, A61P 31/14

(54) **USE OF A COMPOUND SUCH AS PLERIXAFOR FOR TREATING A VIRAL PULMONARY DISEASE**

(71) Applicant: 4Living Biotech, 59000 Lille (FR); Centre national de la recherche scientifique, 75016 Paris (FR); Institut Pasteur de Lille, 59000 Lille (FR); Université de Lille, 59000 Lille (FR)
(72) Inventor: RATTENBACH, Revital, 75004 Paris (FR); BISMUTH, Keren, 75011 Paris (FR); BRETON, Jérome, 59777 Euralille (FR); TROTTEIN, François, 59200 TOURCOING (FR); SENCIO, Valentin, 59000 Lille (FR)
(74) Representative: Icosa

(57) **Abstract**

The present invention relates to a compound of formula (I) or a pharmaceutically acceptable salt and/or hydrate thereof, for use in the treatment of a viral pulmonary disease in a subject, said disease being different from COVID-19, wherein formula (I) is Z-R-A-R'-Y.

## Description

### FIELD OF INVENTION

The present invention relates to the use of a compound for treating a viral pulmonary disease in a subject said disease being different from COVID-19

### BACKGROUND OF INVENTION

Pulmonary diseases are a major cause of morbidity and mortality and their impact is expected to increase in the future. Respiratory viruses are the most common cause of acute and chronic respiratory infections and additionally it is increasingly recognized that respiratory viruses are a major cause of acute exacerbations of chronic pulmonary diseases.

Viral infection can cause an acute respiratory distress syndrome (ARDS). Among the respiratory viruses that can affect the lung and cause ARDS, pandemic viruses head the list, with influenza viruses H5N1 and H1N1 2009 being the most recently identified. However, other viruses can cause severe ARDS. Apart from these pandemic viruses, respiratory viruses are rarely responsible for viral pneumonia, bronchiolitis and ARDS.

Chronic respiratory failure is a serious illness that gets worse over time and can be in the form of a chronic respiratory distress syndrome (CRDS). As the condition increases in severity, people may develop an abnormal heart rhythm, stop breathing, or slip into a coma.

Despite existing treatments, there is still a need for effective treatments for viral pulmonary diseases, and in particular for treatments ARDS and CRDS in patients.

The inventors have surprisingly found that compounds of formula (I) according to the present invention, or a pharmaceutically acceptable salt and/or hydrate thereof, are able to treat viral pulmonary diseases and more particularly diseases leading to an acute or chronic respiratory distress syndromes.

### SUMMARY

The present invention thus relates to a compound of formula (I) or a pharmaceutically acceptable salt and/or hydrate thereof, for use in the treatment of a viral pulmonary disease in a subject, said disease being different from COVID-19,
wherein formula (I) is:

Z-R-A-R'-Y (I)

wherein:
- Z and Y, which are identical or different, represent:
   ∘ an alkyl group having from 1 to 6 carbon atoms, optionally *substituted* by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
   ∘ an aryl or heteroaryl group having from 3 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
   ∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, or
   ∘ a D₁D₂N- group, wherein D₁ and D₂, which may be identical or different, represent:
      ▪ a hydrogen atom, or
      ▪ an alkyl group having from 1 to 6 carbon atoms optionally substituted by at least one hydroxyl group, a halogen atom, a CF₃ group, a CN group, an amine group, or an alkyl, O-alkyl or S-alkyl group having from 1 to 12 carbon atoms, or
      ▪ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF₃ group, CN group, amine group, or alkyl, O-alkyl or S-alkyl group having from 1 to 12 carbon atoms, or
      ▪ D₁ and D₂ are linked together to from a N-containing aryl or heteroaryl group having from 3 to 12 carbon atoms and optionally substituted by at least one amine group optionally substituted by an alkylheteroaryl group having from 3 to 12 carbon atoms, and
- A represents:
   ∘ an aryl or heteroaryl group having from 3 to 12 carbon atoms, or
   ∘ an alkyl group having from 1 to 6 carbon atoms, or
   ∘ -R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF3 group, a CN group or an alkyl, O-alkyl or S-alkyl group having from 1 to 6 carbon atoms optionally substituted by a hydroxyl group, an amine group or an O-alkyl group having from 1 to 6 carbon atoms, and
   - R and R', which are identical or different, represent an alkyl group having from 1 to 6 carbon atoms or a R₁NR₂R₃ group or a single bond, and
   - R₁ represents a single bond or an alkyl group having from 1 to 6 carbon atoms, and
   - R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms

In a more specific aspect, the invention relates to a compound wherein:
- Z and Y, which are identical or different, represent:
   ∘ an alkyl group having from 1 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
   ∘ an aryl or heteroaryl group having from 3 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
   ∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, and
- A represents an aryl or heteroaryl group having from 3 to 12 carbon atoms, and
- R and R', which are identical or different, represent an alkyl group having from 1 to 6 carbon atoms or a R₁NR₂R₃ group, and
- R₁ represents a single bond or an alkyl group having from 1 to 6 carbon atoms, and
- R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms.

According to a further aspect, the invention relates to a compound for use according to claim 1, wherein said compound of formula (I) is represented by the formula (II): 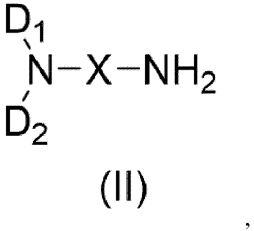 wherein:
- D₁ and D₂, which may be identical or different, represent:
   ∘ an alkyl group having from 1 to 6 carbon atoms optionally substituted by at least one hydroxyl group, a halogen atom, a CF₃ group, a CN group, an amine group, or an alkyl, O-alkyl or S-alkyl group having from 1 to 12 carbon atoms, or
   ∘ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF₃ group, CN group, amine group, or alkyl, O-alkyl or S-alkyl group having from 1 to 12 carbon atoms, or
   ∘ D₁ and D2 are linked together to from a N-containing aryl or heteroaryl group having from 3 to 12 carbon atoms and optionally substituted by at least one amine group optionally substituted by an alkylheteroaryl group having from 3 to 12 carbon atoms, and
- X represents:
   ∘ an alkyl group having from 1 to 6 carbon atoms, or
   ∘ R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF3 group, a CN group or an alkyl, O-alkyl or S-alkyl group having from 1 to 6 carbon atoms optionally substituted by a hydroxyl group, an amine group or an O-alkyl group having from 1 to 6 carbon atoms

In an even more specific aspect, the invention relates to a compound for use for the treatment of a viral pulmonary disease in a subject, wherein said compound is 1,1'-[1,4-phenylene-bis(methylene)]-bis-1,4,8,11-tetraazacyclotetradecane.

In a further aspect of the invention, the compound is for use for the treatment of a viral pulmonary disease in a subject wherein the viral pulmonary disease leads to an acute and/or chronic respiratory distress syndrome.

In a still further aspect, the compound of the invention is for use for the treatment of a viral pulmonary disease in a subject wherein the viral pulmonary disease is caused by a virus chosen among influenza virus, respiratory syncytial virus, parainfluenza viruses, metapneumovirus, rhinovirus, coronaviruses such as severe acute respiratory syndrome coronavirus (SARS-CoV) coronavirus except SARS-CoV2 or Middle East respiratory syndrome coronavirus (MERS-CoV), adenoviruses, and bocaviruses

In a still further aspect, the compound of the invention is for use for the treatment of a viral pulmonary disease in a subject wherein the viral pulmonary disease is chosen among influenza, bronchiolitis, laryngotracheitis, pneumopathy, pneumonia.

The invention also relates to a pharmaceutical composition for use in treating a viral pulmonary disease said disease being different from COVID-19 comprising the compound as above described and a pharmaceutically acceptable excipient.

In a more specific aspect, the pharmaceutical composition for use according to claim 8 further comprising at least one other active agent.

In an even more specific aspect of the invention, the other active agent of the pharmaceutical composition of the invention is an anti-inflammatory selected from the group comprising nonsteroidal anti-inflammatory drugs (NSAIDs) such as Ibuprofen, Naproxen, Celecoxib, Diclofenac, Indomethacin, Oxaprozin, and Piroxicam or steroidal anti-inflammatory drugs such as Cortisone, Dexamethasone, Hydrocortisone, Methylprednisolone or Prednisolone.

In an even more specific aspect of the invention, the other active agent of the pharmaceutical composition of the invention is selected from the group comprising an immune checkpoint inhibitor (ICI), such as an inhibitor of PD-1, an inhibitor of PD-L1, an inhibitor of CTLA-4 and an inhibitor of LAG3, preferably, the active agent is an inhibitor of PD-1 and an inhibitor of PD-L1.

In a specific aspect of the invention, the pharmaceutical composition is administrated in a therapeutically effective amount.

In a further aspect, the pharmaceutical composition of the invention is administrated via parenteral route comprising intravenous, intramuscular, subcutaneous or intradermal route.

In a further aspect, the compound of the pharmaceutical composition of the invention is Plerixafor and Plerixafor is administrated via subcutaneous route at a dosage from 0,24 mg/kg/day to 1,44 mg/kg/day during at least 7 days.

In a further aspect, the compound of the pharmaceutical composition of the invention is Plerixafor and wherein plerixafor is administrated intravenously at a dosage from 10 µg/kg/hour to 80 µg/kg/hour continuously during at least 7 days.

### DEFINITIONS

In the present invention, the following terms have the following meanings:

Within the meaning of the invention "**a**" or "**an**" refer to one or to more than one (i.e., to at least one) of the grammatical object of the article. By way of example, "an element" means one element or more than one element.

Within the meaning of the invention "**about**" is used herein to mean approximately, roughly, around, or in the region of. When the term "about" is used in conjunction with a numerical range, it modifies that range by extending the boundaries above and below the numerical values set forth.

"**Alkyl**" by itself or as part of another substituent refers to a linear, branched or cyclic saturated hydrocarbyl group. Typically, alkyl groups comprise from 1 to 12 carbon atoms, preferably from 1 to 6 carbon atoms, more preferably from 1 to 3 carbon atoms, furthermore preferably 1 to 2 carbon atoms. Alkyl groups may be linear or branched and may be substituted as indicated herein. Non-limiting examples of alkyl groups include methyl, ethyl, *n*-propyl, *i*-propyl, *n*-butyl, *i*-butyl, *s*-butyl and *t*-butyl, pentyl and its isomers (*e.g.*, *n*-pentyl, *iso*-pentyl), and hexyl and its isomers (*e*.*g.*, *n*-hexyl, *iso*-hexyl). Preferred alkyl groups include methyl, ethyl, *n*-propyl, *i*-propyl, *n*-butyl, *s*-butyl and *t-*butyl. "**Heteroalkyl**"**,** in particular "**heterocycloalkyl**"**,** refers to an alkyl, in particular a cycloalkyl, group comprising at least one heteroatom preferably selected from the group consisting of O, P, N, S and Si, which is more preferably N. "**Alkylaryl**" refers to an aryl group substituted by at least one alkyl group. **"Alkoxy"** refers to O-alkyl.

**"Amine"** refers to -NH₂ group. In some embodiments, the amine groups may be substituted, thus providing secondary or tertiary amine groups.

"**Aryl**" refers to a cyclic, polyunsaturated, aromatic hydrocarbyl group comprising at least one aromatic ring. Aryl groups may have a single ring (*i.e.*, phenyl) or multiple aromatic rings fused together (*e.g.,* naphthyl) or linked covalently. Typically, aryl groups have from 5 to 12 carbon atoms, preferably from 6 to 10 carbon atoms. "**Heteroaryl**" refers to an aryl group comprising at least one heteroatom preferably selected from the group consisting of O, P, N, S and Si, which is more preferably N. "**Arylalkyl**" refers to an alkyl group substituted by at least one aryl group.

"**At least one**" refers to one, two, three or more.

"**Halogen**" refers to fluoro, chloro, bromo or iodo. Preferred halogen groups include fluoro and chloro.

**"Hydrate of a compound"** refers to a molecular complex comprising the compound and one or more pharmaceutically acceptable solvent molecules, wherein the solvent is water.

**"Plerixafor^{®}"** or "plerixafor" refers to a bicyclam derivative which chemical name is 1,1'-[1,4-phenylene-bis(methylene)]-bis-1,4,8,11-tetraazacyclotetradecane. Plerixafor is also designated under the name of AMD3100 and its CAS reference number is 110078-46-1.

**"Salt of a compound"** refers to acid addition or base salts of said compound. Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts. Suitable base salts are formed from bases which form non-toxic salts. Examples include the aluminium, arginine, benzathine, calcium, choline, diethylamine, 2-(diethylamino)ethanol, diolamine, ethanolamine, glycine, 4-(2-hydroxyethyl)-morpholine, lysine, magnesium, meglumine, morpholine, olamine, potassium, sodium, tromethamine and zinc salts. In one embodiment, the salt is selected from the group consisting of hydrobromide, hydrochloride, dihydrobromide, dihydrochloride, zinc and copper salts.

Within the meaning of the invention the **"subject"** is an animal, preferably a mammal, more preferably a human, receiving the composition of the invention. In the sense of the present invention, a subject may be a patient, i.e. a person receiving medical attention, undergoing or having underwent a medical treatment, or monitored for the development of a disease.

### DETAILED DESCRIPTION

The present invention relates to a compound of formula (I) or a pharmaceutically acceptable salt and/or hydrate thereof, for use in the treatment of a viral pulmonary disease.

According to the present invention, the viral pulmonary disease is different from COVID-19.

Formula (I) is:

Z-R-A-R'-Y (I),

wherein :
- Z and Y, which are identical or different, represent:
   o an alkyl group having from 1 to 6 carbon atoms, optionally substituted by a hydroxyl group, a halogen atom, a CN group, a CF3 group, a SO group, a NO2 group, an amine group, a difluor or an alkyl, S-alkyl or O-alkyl group having from 1 to 6 carbon atoms, or
   o an aryl or heteroaryl group having from 3 to 6 carbon atoms, optionally substituted by a hydroxyl group, a halogen atom, a CN group, a CF3 group, a SO group, a NO2 group, an amine group, a difluor or an alkyl, S-alkyl or O-alkyl group having from 1 to 6 carbon atoms, or
   ∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, or
   ∘ a D₁D₂N- group, wherein D₁ and D₂, which may be identical or different, represent :
      ▪ a hydrogen atom, or
      ▪ an alkyl group having from 1 to 6 carbon atoms optionally substituted by at least one hydroxyl group, a halogen atom, a CF₃ group, a CN group, an amine group, or an alkyl, O-alkyl or S-alkyl group having from 1 to 12 carbon atoms, or
      ▪ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF₃ group, CN group, amine group, or alkyl, O-alkyl or S-alkyl group having from 1 to 12 carbon atoms, or
      ▪ D₁ and D₂ are linked together to from a N-containing aryl or heteroaryl group having from 3 to 12 carbon atoms and optionally substituted by at least one amine group optionally substituted by an alkylheteroaryl group having from 3 to 12 carbon atoms, and
- A represents :
   ∘ an aryl or heteroaryl group having from 3 to 12 carbon atoms, or
   ∘ an alkyl group having from 1 to 6 carbon atoms, or
   ∘ -R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF₃ group, a CN group or an alkyl, O-alkyl or S-alkyl group having from 1 to 6 carbon atoms optionally substituted by a hydroxyl group, an amine group or an O-alkyl group having from 1 to 6 carbon atoms, and
- R and R', which are identical or different, represent an alkyl group having from 1 to 6 carbon atoms or a R₁NR₂R₃ group or a single bond, and
- R₁ represents a single bond or an alkyl group having from 1 to 6 carbon atoms, and
- R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms.

In one embodiment, Formula (I) is:

Z-R-A-R'-Y (I),

wherein :
- Z and Y, which are identical or different, represent:
   ∘ an alkyl group having from 1 to 6 carbon atoms, optionally substituted by a hydroxyl group, a halogen atom, a CN group, a CF₃ group, a SO group, a NO2 group, an amine group, a difluor or an alkyl, S-alkyl or O-alkyl group having from 1 to 6 carbon atoms, or
   ∘ an aryl or heteroaryl group having from 3 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
   ∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, and
- A represents an aryl or heteroaryl group having from 3 to 12 carbon atoms, and
- R and R', which are identical or different, represent an alkyl group having from 1 to 6 carbon atoms or a R₁NR₂R₃ group, and
- R₁ represents a single bond or an alkyl group having from 1 to 6 carbon atoms, and
- R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms.

In an embodiment, the compound is of formula (I), wherein:
- Z and Y are identical and represent cyclic polyamine moieties having from 9 to 20 ring members and from 3 to 6 amine groups in the ring spaced by 2 or more carbon atoms from each other, and
- A represents an aryl or heteroaryl group having from 3 to 8 carbon atoms, and
- R and R', which are identical or different, represent a methylene linked to a nitrogen atom of Z or Y, the nitrogen atoms being otherwise unsubstituted.

In one embodiment, Z and/or Y is a cyclic polyamine containing 9-32 ring members of which 3-8 are nitrogen atoms, said nitrogen atoms being separated from each other by at least 2 carbon atoms. Said cyclic polyamine may contain 10-20 ring members, preferably 14 or 16 ring members, more preferably 14 ring members. Said cyclic polyamine may contain 3-6 nitrogen atoms, preferably 4 or 5 nitrogen atoms, more preferably 5 nitrogen atoms. Said nitrogen atoms may be separated from each other by at least 2 carbon atoms, in particular by 2 or 3 carbon atoms. Said cyclic polyamine may contain additional heteroatoms besides nitrogen, in particular oxygen or sulfur, and/or may be fused to an additional ring system, in particular to a pyridine.

In particular, A may be selected from the group consisting of phenylene, pyridine, thiophene, pyrazine and imidazole.

In one embodiment, the compound of formula (I) is selected from the group consisting of the compounds represented by the following structures:

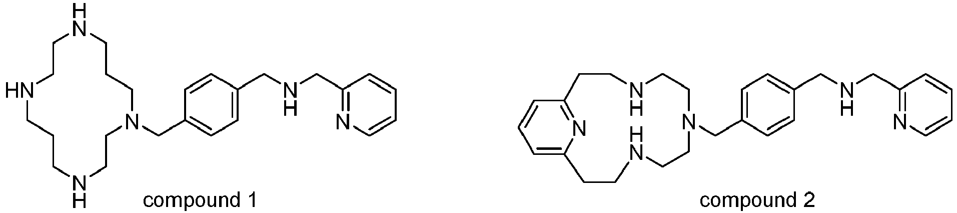

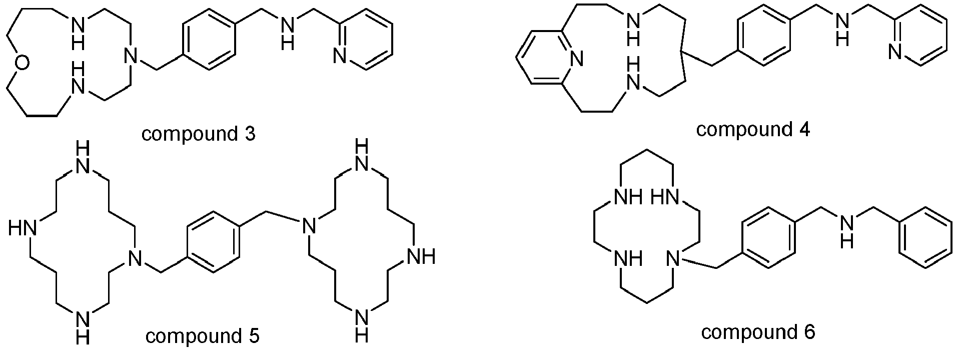

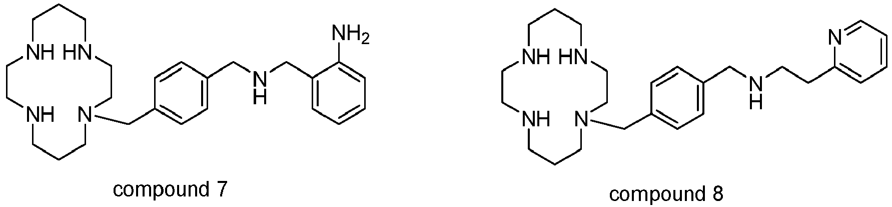

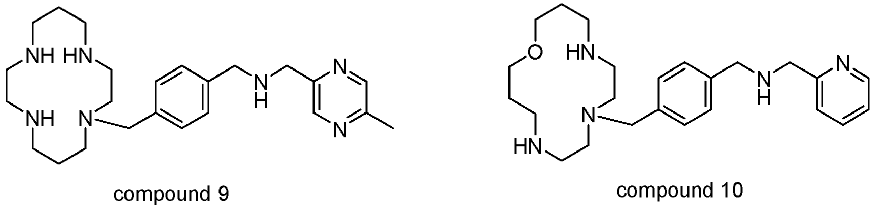

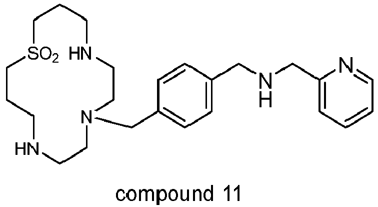

In one preferred embodiment, the compound of formula (I) is 1,1'-[1,4-phenylene-bis(methylene)]-bis-1,4,8,11-tetraazacyclotetradecane, also named Plerixafor or AMD31000.

In one embodiment, the compound of formula (I) is represented by the following formula (II): 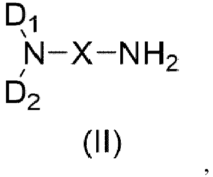 wherein :
- D₁ and D₂, which may be identical or different, represent:
   ∘ an alkyl group having from 1 to 6 carbon atoms optionally substituted by at least one hydroxyl group, a halogen atom, a CF₃ group, a CN group, an amine group, or an alkyl, O-alkyl or S-alkyl group having from 1 to 12 carbon atoms, or
   ∘ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF₃ group, CN group, amine group, or alkyl, O-alkyl or S-alkyl group having from 1 to 12 carbon atoms, or
   ∘ D₁ and D₂ are linked together to from a N-containing aryl or heteroaryl group having from 3 to 12 carbon atoms and optionally substituted by at least one amine group optionally substituted by an alkylheteroaryl group having from 3 to 12 carbon atoms, and
- X represents:
   ∘ an alkyl group having from 1 to 6 carbon atoms, or
   ∘ -R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF₃ group, a CN group or an alkyl, O-alkyl or S-alkyl group having from 1 to 6 carbon atoms optionally substituted by a hydroxyl group, an amine group or an O-alkyl group having from 1 to 6 carbon atoms.

In an embodiment, Y' is selected from the group consisting of phenylene, pyridine and thiophene.

In one embodiment, the compound of formula (I) is represented by the above-mentioned formula (II), wherein:
- D₁ and D₂, which may be identical or different, represent an aryl or heteroaryl group having from 3 to 12 carbon atoms, optionally substituted by a hydroxyl group or an alkyl group having from 1 to 6 carbon atoms, and
- X represents an alkyl group having from 1 to 6 carbon atoms.

In particular, D₁ may be a monocyclic or bicyclic fused ring system containing at least one nitrogen atom, for example a 5,6,7,8-tetrahydroquinoline.

In particular, D₂ may be a monocyclic or fused bicyclic unsubstituted or substituted ring system containing at least one heteroatom selected from N, O and S. D₂ may be selected from the group consisting of substituted or unsubstituted dihydroquinoline, tetrahydroquinoline, pyranopyridine, dihydropyranopyridine, thiapyranopyridine, dihydrothiapyranopyridine, dihydronaphthyridine, tetrahydronaphthyridine, imidazole, oxazole, thiazole, benzimidazole, benzothiazole, and benzoxazole.

In one embodiment, the compound of formula (I) is represented by the following formula (III): 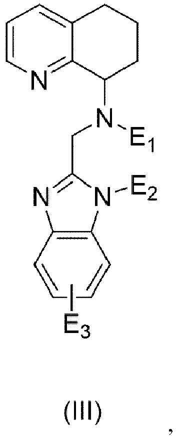 wherein :
- E₁ represents an alkyl group having from 1 to 12 carbon atoms, or a heteroaryl group having from 3 to 12 carbon atoms, and
- E₂ represents a heteroalkyl group having from 1 to 12 carbon atoms, substituted by an amine group, and
- E₃ represents a heteroalkyl group having from 1 to 12 carbon atoms.

In one embodiment, the compound of formula (I) is selected from the group consisting of the compounds represented by the following structures:

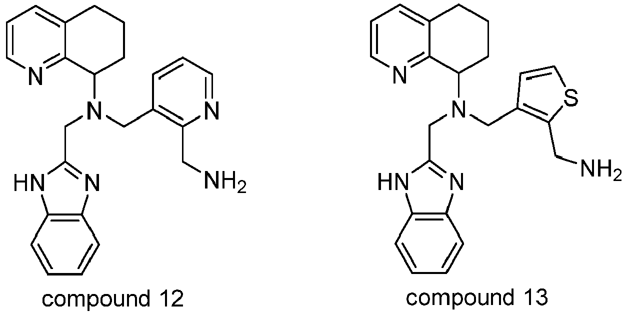

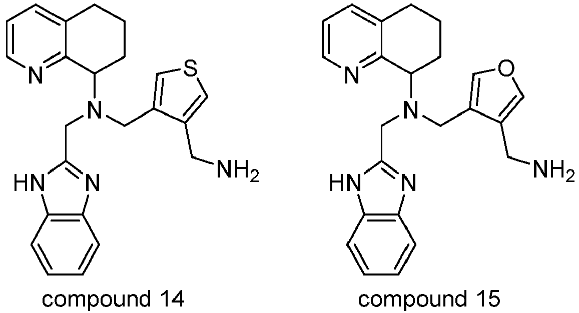

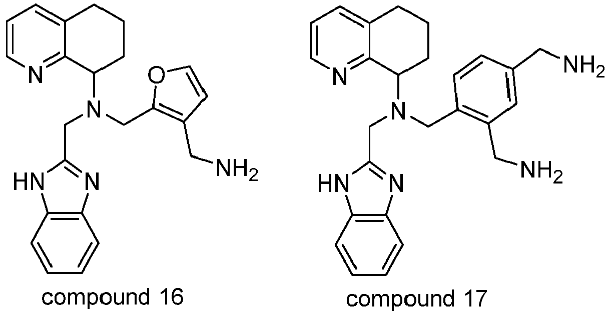

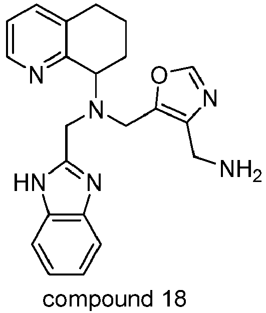

For example, the compound of formula (I) may be N'-(1H-benzimidazol-2-ylmethyl)-N'-[(8S)-5,6,7,8-tetrahydroquinolin-8-yl]butane-1,4-diamine, also named AMD070 or mavorixafor.

In one embodiment, the compound for use according to the invention is selected from the group consisting of an indole-based compound, a N-substituted indole compound, a bicyclam compound, a cyclam mimetic compound, a paraxylylenediamine-based compound, a guanidine-based compound, a tetrahydroquinoline-based compound, and a 1,4-phenylenebis(methylene) compound.

According to a preferred embodiment, the compound for use according to the invention is selected from the group consisting of plerixafor (AMD3100), Burixafor (TG-0054), JM1657, AMD3329, AMD3465, mavorixafor (AMD070), MSX-122, CTCE-9908, WZ811, and BKT-140.

In a particularly preferred embodiment, the compound for use according to the invention is 1,1'-[1,4-phenylene-bis(methylene)]-bis-1,4,8,11-tetraazacyclotetradecane, also named Plerixafor or AMD31000.

Within the meaning of the invention, "treating" or "treatment" or "alleviation" refers to both therapeutic treatment and prophylactic or preventative measures; wherein the object is to prevent or slow down (lessen) the targeted pathologic condition or disorder. Those in need of treatment include those already with the disorder as well as those prone to have the disorder or those in whom the disorder is to be prevented. A subject or mammal is successfully "treated" for an infection if, after receiving a therapeutic amount of an antibody according to the methods of the present invention, the patient shows observable and/or measurable reduction in or absence of one or more of the following: reduction in the number of pathogenic cells; reduction in the percent of total cells that are pathogenic; and/or relief to some extent, one or more of the symptoms associated with the specific disease or condition; reduced morbidity and mortality, and improvement in quality of life issues. The above parameters for assessing successful treatment and improvement in the disease are readily measurable by routine procedures familiar to a physician.

In a further embodiment the compound of the invention is for use in treating a viral pulmonary disease leading to acute and/or chronic respiratory distress syndrome.

Within the meaning of the invention, an acute respiratory distress syndrome (ARDS) is recognized as the most severe form of acute lung injury (ALI), a form of diffuse alveolar injury. The American-European Consensus Conference (AECC) defined ARDS as an acute condition characterized by bilateral pulmonary infiltrates and severe hypoxemia in the absence of evidence for cardiogenic pulmonary edema.

Chronic respiratory failure, however, is an ongoing condition. It gradually develops over time and requires long-term treatment. Chronic respiratory failure usually happens when the airways that carry air to the lungs become narrow and damaged. Chronic respiratory failure can also be classified as hypoxemic or hypercapnic respiratory failure. Low blood oxygen levels cause hypoxemic respiratory failure. High carbon dioxide levels cause hypercapnic respiratory failure. In a specific embodiment of the invention, the chronic respiratory failure is a respiratory distress syndrome.

In a specific embodiment, the compound of the invention is for use in treating a viral pulmonary disease caused by influenza virus, respiratory syncytial virus, parainfluenza viruses, metapneumovirus, rhinovirus, coronaviruses such as severe acute respiratory syndrome coronavirus (SARS-CoV) coronavirus except SARS-CoV2 or Middle East respiratory syndrome coronavirus (MERS-CoV), adenoviruses, and bocaviruses.

In a further embodiment the compound of the invention is for use for treating a viral pulmonary disease chosen among influenza, bronchiolitis, laryngotracheitis, pneumopathy, pneumonia.

The present invention also relates to the use of a compound of formula (I) as described above or a pharmaceutically acceptable salt and/or hydrate thereof for the manufacture of a medicament for the treatment of a viral pulmonary disease as described above.

The present invention also relates to the use of a compound of formula (I) as described above or a pharmaceutically acceptable salt and/or hydrate thereof for the treatment of a viral pulmonary disease as described above.

The present invention also relates to a method for treating a viral pulmonary disease as described above, comprising the administration to a subject in need thereof of a therapeutically effective amount of a compound of formula (I) according to the invention.

In a specific embodiment, the invention relates to a pharmaceutical composition for use in the treatment of a viral lung (pulmonary) disease, said disease being different from COVID-19, said pharmaceutical composition comprising a compound of formula (I) as described above or a pharmaceutically acceptable salt and/or hydrate thereof, and a pharmaceutically acceptable excipient.

Within the meaning of the invention the expression "pharmaceutical composition" refers to a composition comprising an active principle in association with a pharmaceutically acceptable vehicle or excipient. A pharmaceutical composition is for therapeutic use, and relates to health. Especially, a pharmaceutical composition may be indicated for treating or preventing a disease.

Within the meaning of the invention the expression "pharmaceutically acceptable excipient" refers to an inert vehicle or carrier used as a solvent or diluent in which the pharmaceutically active agent is formulated and/or administered, and which does not produce an adverse, allergic or other reaction when administered to an animal, preferably a human. This includes all solvents, dispersion media, coatings, antibacterial and antifungal agents, isotonic agents, absorption retardants and the like. For human administration, preparations must meet standards of sterility, general safety and purity as required by regulatory agencies, such as the FDA or EMA.

In a further embodiment of the invention, the pharmaceutical composition is administrated in a therapeutically effective amount.

Within the meaning of the invention the expression "therapeutically effective amount" means level or amount of compound that is aimed at, without causing significant negative or adverse side effects to the subject, (1) delaying or preventing the onset of a disease, disorder, or condition; (2) slowing down or stopping the progression, aggravation, or deterioration of one or more symptoms of the disease, disorder, or condition; (3) bringing about ameliorations of the symptoms of the disease, disorder, or condition; (4) reducing the severity or incidence of the disease, disorder, or condition; or (5) curing the disease, disorder, or condition. A therapeutically effective amount may be administered prior to the onset of the disease, disorder, or condition, for a prophylactic or preventive action. Alternatively, or additionally, the therapeutically effective amount may be administered after initiation of the disease, disorder, or condition, for a therapeutic action.

Within the meaning of the invention the term "administration", or a variant thereof (e.g. "administering"), means providing the active agent or active ingredient, alone or as part of a pharmaceutically acceptable composition, to the subject in whom/which the condition, symptom, or disease is to be treated or prevented.

In a further embodiment of the invention, the pharmaceutical composition is administrated by via parenteral route comprising intravenous, intramuscular, subcutaneous or intradermal route.

In a further embodiment of the invention, the pharmaceutical composition of the invention is injected. Within the meaning of the invention the expression "injectable composition" means a composition suitable for being introduced in animal, including human, body or its parts, for example but not exclusively by intradermal, hypodermal, intramuscular, intravenous, or epidural administration routes. It is sterile.

In a more specific embodiment of the invention, the pharmaceutical composition comprises Plerixafor.

In a still further specific aspect of the invention, the pharmaceutical composition comprising Plerixafor is administrated via subcutaneous route at a dosage from 0.20 mg/kg/day to 1.50, mg/kg/day preferably from 0,24 mg/kg/day to 1,44 mg/kg/day.

In a still further specific aspect of the invention, the pharmaceutical composition comprising Plerixafor is administrated intravenously at a dosage from 10 µg/kg/hour to 80 µg/kg/hour preferably continuously.

In a still further specific aspect of the invention, the pharmaceutical composition comprising Plerixafor is administrated during at least 1, 2, 3, 4, 5, 6, 7, 8 9 or 10 days, preferably during 7 days.

In a still further specific aspect of the invention, the pharmaceutical composition comprising Plerixafor is administrated via subcutaneous route at a dosage from 0,24 mg/kg/day to 1,44 mg/kg/day during 7 days.

In a still further specific aspect of the invention, the pharmaceutical composition comprising Plerixafor is administrated intravenously at a dosage from 10 µg/kg/hour to 80 µg/kg/hour continuously during 7 days.

In a further embodiment, the invention relates to the pharmaceutical composition comprising the compound as above described and further comprises an active agent.

According to the meaning of the invention, said further active agent is different from the compound of the invention.

According to a specific aspect of the invention, the active agent is an anti-inflammatory selected from the group comprising nonsteroidal anti-inflammatory drugs (NSAIDs) such as Aspirin, Naproxene, Celecoxib, Diflunisal, Etodolac, Ibuprofen, Indomethacin, Ketoprofen, Ketorolac, Nabumetone, Oxaprozin, Piroxicam, Salsalte, Sulindac, Tolmetin.

According to a specific aspect of the invention, the active agent is an anti-inflammatory selected from the group comprising steroidal anti-inflammatory drugs such as Bethamethasone, Cortisone, Dexamethasone, Hydrocortisone, Triamcinolone acetonide, Methylprednisolone, or Prednisolone.

According to a specific aspect of the invention, the active agent is oxygen.

In a further embodiment, the invention relates to a pharmaceutical composition further comprising an active agent wherein the active agent is selected from the group comprising an immune checkpoint inhibitor (ICI), such as an inhibitor of PD-1, an inhibitor of PD-L1, an inhibitor of CTLA-4 and an inhibitor of LAG3,

In a further embodiment, the invention relates to a pharmaceutical composition further comprising an active agent wherein the active agent an inhibitor of PD-1 and an inhibitor of PD-L1.

### BRIEF DESCRIPTION OF THE DRAWINGS

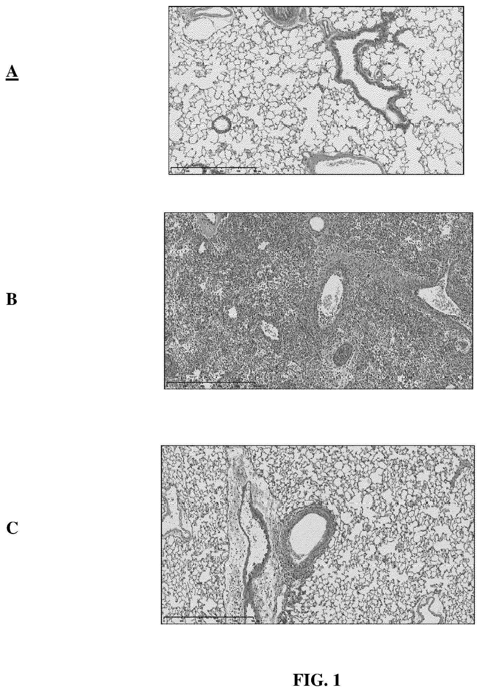

**Figure 1A-1C** is a photograph showing pulmonary lobe section from mice stained with Hematoxylin & Eosin (H&E) and digitized using a Nanozoomer from Hamamatsu at Sciempath laboratory of non-infected mice, i.e. Group 1M (Fig. 1A), influenza treated mice, i.e. Group 2M (Fig. 1B) and influenza treated mice + Plerixafor (20 mg/kg) treated mice, i.e. Group 6M (Fig. 1C).

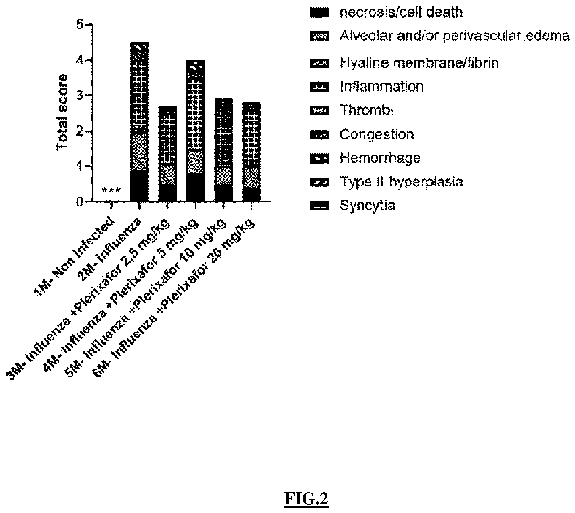

**Figure 2** is a graph showing Total score according Meyerholz and Beck (Mean ± SEM; n=10 (or 6 for non-infected vehicle group) ***p<0.001 in comparison to group 2M) of non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg). This score take account of 9 parameters: cellular death/necrosis, alveolar and/or perivascular edema, hyaline membrane or fibrin, congestion, inflammation, thrombi, hemorrhage, type II hyperplasia, and syncytia.

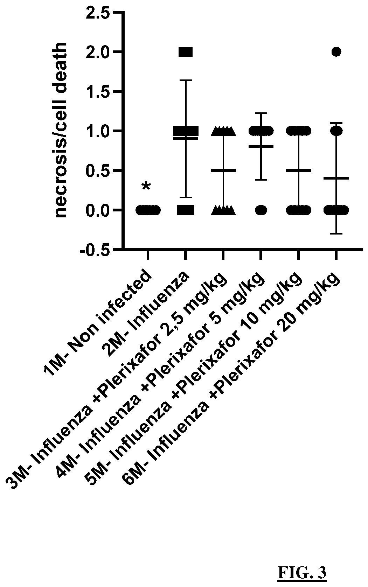

**Figures 3** is a representation of Necrosis cell death score (Mean ± SEM; n=10 (or 6 for non-infected vehicle group) *p<0.05 in comparison to group 2M) of non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg)

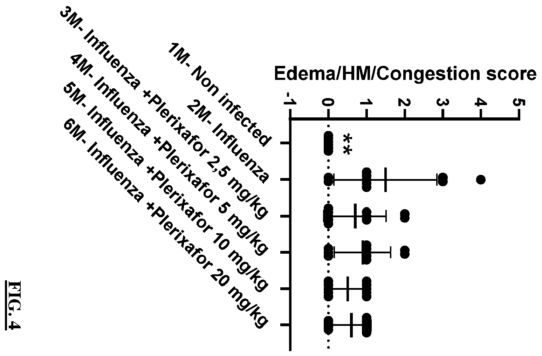

**Figure 4** is a representation of Hyaline membrane and congestion score (Mean ± SEM; n=10 (or 6 for non-infected vehicle group) **p<0.01 in comparison to group 2M) of non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg)

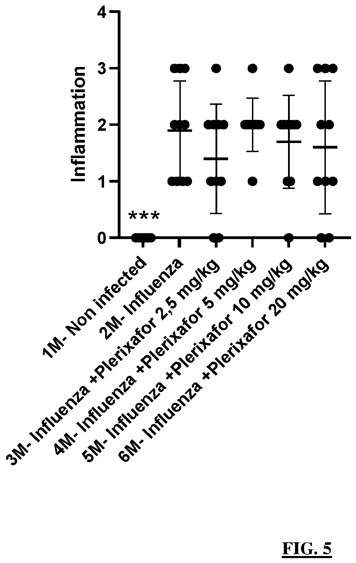

**Figure 5** is a representation of Inflammation score (Mean ± SEM; n=10 (or 6 for non-infected vehicle group) ***p<0.001 in comparison to group 2M) of non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

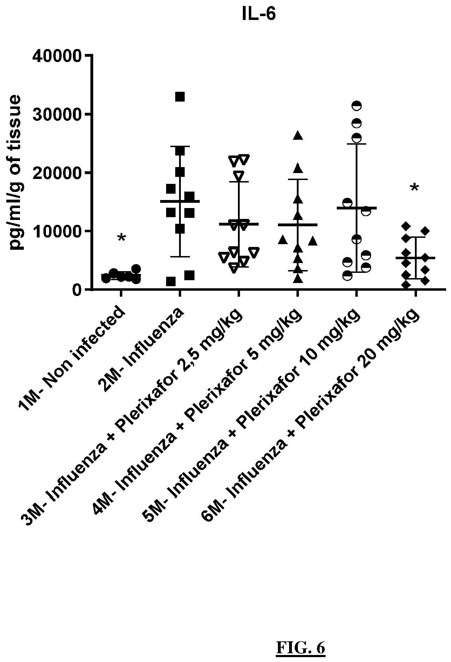

**Figure 6** is a graph representing the concentration of IL-6 in lung homogenates (in pg/ml/g of tissue; Mean ± SEM; n=10 (or 6 for non-infected vehicle group) *p<0.05 in comparison to group 2M) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

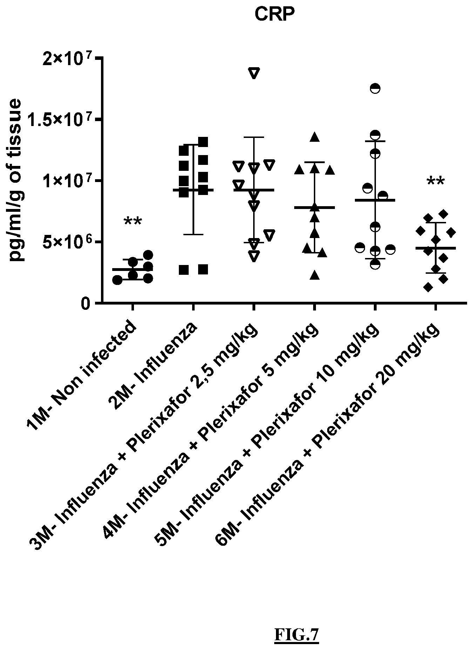

**Figure 7** is a graph representing the concentration of CRP in lung homogenates (in pg/ml/g of tissue; Mean ± SEM; n=10 (or 6 for non-infected vehicle group) **p<0.01 in comparison to group 2M) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

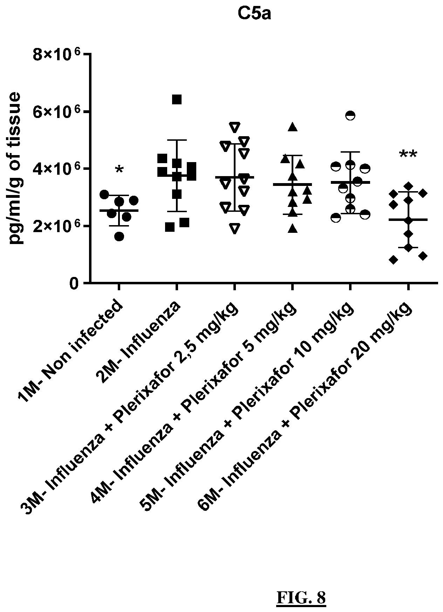

**Figure 8** is a graph representing the concentration of C5a in lung homogenates (in pg/ml/g of tissue; Mean ± SEM; n=10 (or 6 for non-infected vehicle group) *p<0.05, **p<0.01 in comparison to group 2M) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

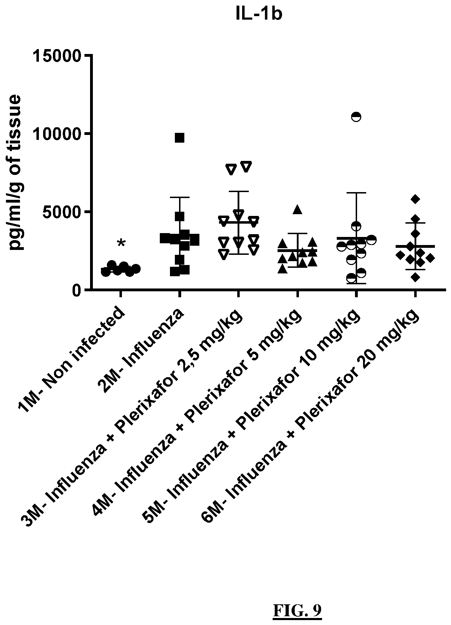

**Figure 9** is a graph representing the concentration of IL-1□ in lung homogenates (in pg/ml/g of tissue; Mean ± SEM; n=10 (or 6 for non-infected vehicle group) *p<0.05 in comparison to group 2M) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

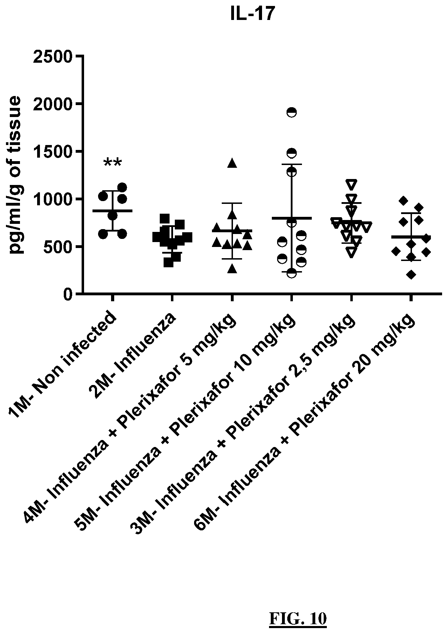

**Figure 10** is a graph representing the concentration of IL-17 in lung homogenates (in pg/ml/g of tissue; Mean ± SEM; n=10 (or 6 for non-infected vehicle group) **p<0.01 in comparison to group 2M) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

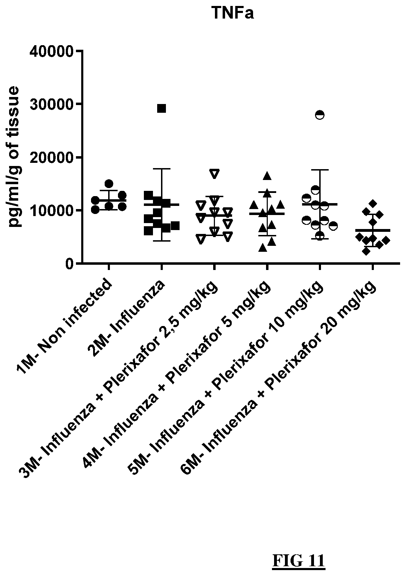

**Figure 11** is a graph representing the concentration of TNF□ in lung homogenates (in pg/ml/g of tissue; Mean ± SEM; n=10 (or 6 for non-infected vehicle group)) in non-infected mice (1M - Non infected), infected mice (2M-influenza), infected mice treated with 2.5 mg/kg of Plerixafor (3M-Influenza+Plerixafor 2.5 mg/kg), infected mice treated with 5 mg/kg of Plerixafor (4M-Influenza+Plerixafor 5 mg/kg), infected mice treated with 10 mg/kg of Plerixafor (5M-Influenza+Plerixafor 10 mg/kg), infected mice treated with 20 mg/kg of Plerixafor (6M-Influenza+Plerixafor 20 mg/kg).

### EXAMPLES

The present invention is further illustrated by the following examples.

### Example 1: Materials and Methods

Influenza A virus infection and vehicle: H3N2 influenza A virus strain Scotland/20/74 (supplied by Institut Pasteur de Lille).
Plerixafor (AMD31000) (Sigma or Bio-techne)
Elisa material: Precellys lysing kit (hard tissue homogenizing CK28, Bertin); RIPA Buffer (Sigma)., Complete tablets mini EASYpack (Roche), Mouse C- Reactive (CRP) Duoset, Mouse complement C5a Duoset,, Mouse CXCL1/KC Duoset, TNF□ (R&D Systems), IL-6 , IL-1b, IL-17 (In vitrogen)

### H3N2 influenza A virus infection:

H3N2 virus stock solution was diluted in PBS to obtain 50 PFU in 50µl. H3N2 was administered intranasally to mice from group 2M to 6M (50µl). Mice from group 1M received PBS (50µl).

### Items formulation for treatment:

Water for injection (vehicle) was supplied as "ready to use" for subcutaneous (SC) injection (100µl) for group 2M.

Plerixafor^{®} supplied as powder was weighted and diluted adequately in water to inject twice a day 1.25, 2.5, 5 and 10 mg/kg subcutaneously (100 µl) for group 3M, 4M, 5M and 6M, respectively.

### EXPERIMENTAL MODEL

### Animals

Species / Strain: Mice / C57BL6/J
Gender / Number / Age: Males / 9 weeks
Source: Janvier Labs, France.
Initial Body weight: Mean body weight was 25.03 ± 0.8 g. The minimum and maximum weight of each group did not exceed ±20 % of group mean weight.
Acclimation period: Not less than three (3) days
Identification: Colors markers on the tail and cage cards.
Animal Management: Housing

Animal handling was performed according to guidelines of the Federation of European Laboratory Animal Science Associations (FELASA).
Diet: Animals were fed ad libitum a commercial rodent diet (Safe ref #A04). Animals had free access to filtered drinking osmotic water.
Contaminants: There were no contaminants in food and water supplies that have the potential to influence the outcomes of this test.
Environment conditions: Animals were housed under standard laboratory conditions, air filtered with adequate fresh air supply. Animals were kept in a climate-controlled environment. Temperatures range was between 20-240C and relative humidity between 30-70% with 12 hours light and 12 hours dark cycle.
Randomization: Mice were allocated to cages randomly upon receipt.

As procedures were performed with H3N2 virus, animal experimentation was performed in an animal biosafety level 2.
Veterinary care: Animals were inspected on arrival in order to fit for the study. Animals were inspected daily for any signs of morbidity of mortality.
Ethics Committee: This study was performed in compliance with "The French Animal Welfare Act" and following "The French Board for Animal Experiments". This study was performed under approval of the French "Ministère de l'enseignement supérieur et de la recherche" (ethics committee n°CEEA75) in compliance with the "Directive 2010/63/UE".

### EXPERIMENTAL DESIGN AND CONDITIONS

Humane end points (i.e. the earliest indicator in an animal experiment of (potential) pain and/or distress that can be used to avoid or limit pain and/or distress): No Animal found in moribund condition.
Study initiation definition: IAV infection day was defined in this study as "DAY 0".
IAV procedure: All procedures were performed under biosafety cabinet 2.

For the viral infection, mice were anesthetized by intraperitoneal injection of 1.25 mg of ketamine plus 0.25 mg of xylazine in 200 µl of phosphate buffered saline (PBS). Then, mice were intranasally infected with 50 µl of PBS containing 50 plaque forming units (PFU) of the H3N2 virus.

Animals were infected once on day 0 (groups 2M, 3M, 4M, 5M and 6M). For group 1M (non-infected), 50µl of PBS was intranasally administered.

### Treatment administration:

Treatments were administered twice a day SC in mice from day 1 to day 6 by grasping the skin along back of mice. The needle was inserted at base of skin old between thumb and finger and solution was injected. Plerixafor^{®} was administered (100 µl) twice a day to group 3M (1.25 mg/kg), 4M (2.5 mg/kg), 5M (5 mg/kg) and 6M (10 mg/kg). Thus, mice received daily 2.5, 5, 10 and 20mg/kg for 6 days for group 3M, 4M, 5M and 6M respectively. Group 2M received water twice a day (100µl).

### Study Design and Timeline

The study was conducted in two cycles (53 mice per cycles) according to **Table 1** for study design and **Table 2** for study timeline.

**Table 1**

| **Group** | **N=** | **IAV (Intranasal injection of 50µl - 50 PFU)** | **Treatment** | **Dose level** | **Dose volume** | **ROA** | **Dose regimen** |
|---|---|---|---|---|---|---|---|
| 1M | 6 | PBS | - | - | - | - | - |
| 2M | 20 | √ | Vehicle (water for injection) | - | 100 µl | SC | Twice a day |
| 3M | 20 | √ | Plerixafor^{®} | 1.25 mg/kg | | | |
| 4M | 20 | √ | | 2.5 mg/kg | | | |
| 5M | 20 | √ | | 5 mg/kg | | | |
| 6M | 20 | √ | | 10 mg/kg | | | |

**Table 2**

| **Study Day/week*** | **Procedure** | **Remarks** |
|---|---|---|
| **D0** | BW and IAV infection, 50 PFU in 50 | Except for group 1M (Non-infected), 50µl injectable PBS, IN |
| **D1 to D6** | BW and treatment (Vehicle, Plerixafo | SC injection twice a day, 100µ |
| **D7** | BW and termination | 10 mice per groups: lungs collection for histology, protein |

### TESTS AND EVALUATIONS

Morbidity and mortality observation: Morbidity and mortality was performed daily during the study.

Body weight: Body weight (total of 8 measurements) was recorded before study initiation and every day until study termination according to 4P-Pharma's SOP No. 010 "Weighing Laboratory Animals".

Animals sacrifice, blood and tissue collection: On day 7, mice from all groups were euthanized by cervical dislocation.

For 10 mice per groups, the left lung lobe was inflated, harvested and fixed in formaldehyde 4% for histological analysis. A right lung lobe was harvested, weighted, snap frozen and kept at -70°C for protein extraction. Another right lung lobe was harvested, snap frozen and kept at-70°C for ARN extraction.

### Histological analysis:

Lungs were fixed in Formaldehyde 4% for 24h. Then, lungs were rinsed in PBS, transferred in ethanol and sent to a subcontractor (SCIEMPATH LABO) for histological processing and analysis.

Pulmonary lobe section from mice were stained with Hematoxylin & Eosin (H&E) and digitized using a Nanozoomer from Hamamatsu at Sciempath laboratory.

A dual histopathology scoring system adapted from Imai et al. and Meyerholz and Beck (Ref. 14 and 15) were used to assess pulmonary changes in mice. A total of nine variables were qualitatively assessed and ranked with a score from 0 to 4: (1) cellular death/necrosis, (2) alveolar and/or perivascular edema, (3) hyaline membrane or fibrin, (4) inflammation, (5) thrombi, (6) congestion, (7) hemorrhage, (8) type II hyperplasia, and (9) syncytia. For each criterion, a score "0" = absent, "1" = 1-10% of lung section, "2" =11-25% of lung section, "3" =26-50% of lung section, and "4" =>50% of lung section affected.

### ELISA on Lung homogenates

Lungs for protein extraction were cut in small parts and transferred in CK28 lysis tubes containing 500µl of extraction buffer (RIPA Buffer with protease inhibitor cocktail)

Samples were grinded using Precelyss homogenizer with 3 cycles of agitation at 5500 rpm during 10 seconds. Samples were put on ice, transferred in Eppendorf tubes and centrifugated at 11 000g for 5 minutes. Supernatants were collected in a new Eppendorf tubes and kept at -70°C until analysis.

ELISA were performed according to manufacturer's instruction to quantify the production of Interleukin-6 (IL-6), C-Reactive Protein (CRP), Complement component 5a (C5a), Chemokine (C-X-C motif) Ligand 1 (CXCL1), IL-1β, IL-17 and Tumor Necrosis Factor α (TNFα) in lung homogenates. Samples were diluted at 1:000 for CRP and C5a, 1:10 for IL-6 and CXCL1 and 1:3 for IL-1β, IL-17 and TNFα.

Concentration of cytokines in pg/ml were relativized to the corresponding lung weight to present results in pg/ml/g of tissue.

### Statistical analysis

For statistics, we applied a sequential testing strategy reflecting experimental expectations, while retaining the central Mann-Whitney (non-parametric) test. First, we compared the difference between non-infected group (1M) and influenza group (2M). If the difference was found significant, we then compared the difference between Influenza + Plerixafor 20 mg/kg-treated animals (the highest dose of Plerixafor, group 6M) versus Influenza animals (group 2M). If the results were found significant, we tested the statistical difference versus Influenza for the lower tested doses of Plerixafor, sequentially and in descending order (i.e. group 5M, 4M, 3M), until no significant difference was observed.

### Example 2: Morbidity and mortality observation

No animal was found dead or in moribund condition during the study. Influenza infection induced a progressive and moderate BW loss in comparison to non-infected mice (94 ± 5% for influenza treated mice vs 103 ± 4% for non-infected mice, p<0,001 at day 7). Treatment with Plerixafor at doses inferior or equal to 10 mg/kg did not modulate BW loss. However, the dose of 20 mg/kg of Plerixafor significantly reduced BW loss 7 days after the infection (97 ± 5% for Influenza + Plerixafor 20 mg/kg vs 94 ± 5 % for influenza group, p<0,05).

### Example 3: Histological analysis

Lung were collected at termination on day 7 for 10 mice per group for groups 2M to 6M (as the study was performed by cycles, 6 lungs in the cycles 1 and 4 lungs in the cycles 2 were collected) and 6 mice for group 1M (3 lungs in the 2 cycles). Histological analysis was performed on H&E sections by SCIEMPATH LABO. As expected, lungs sections of the six non-infected animals (group 1M) were normal and free of any lesions. In influenza treated mice (group 2M), animals presented lesions characteristic of a subacute and mild to moderate broncho-interstitial viral pneumonia with presence of minimal to mild bronchiolar epithelial necrosis, peri-vascular edema, minimal to moderate multifocal bronchiolo-alveolar and peri-vascular mixed cell (neutrophilic, histiocytic, and lymphocytic) inflammation, and discrete vascular congestion and alveolar hemorrhages in some of the animals. Noteworthy, some heterogeneity was observed mainly in the second cycle with some animals which presented only very minimal and focal perivascular or bronchiolar mixed cell inflammation was noticed (Meyerholz scores = 1). Influenza infected mice treated with Plerixafor (group 3M, 4M, 5M and 6M) presented similar pulmonary lesions as observed in the group 2, however, their severity, distribution and incidence were slightly lower in comparison to influenza treated mice. Representative photographs of H&E lungs sections are presented in **Figure 1****.**

Histological total scores were calculated according Meyerholz and Beck and presented in **Table 1** and **Figure 2****.** This score take account of 9 parameters: cellular death/necrosis (individual parameter presented in **Table 2** and **Figure 3**), alveolar and/or perivascular edema, hyaline membrane or fibrin, congestion (the 3 last parameters were cumulated and presented in **Table 3** and **Figure 4** according to Meyerholz and Beck, 2020), inflammation (individual parameter presented in **Table 4** and **Figure 5**), thrombi, hemorrhage, type II hyperplasia, and syncytia.

Globally, treatments with Plerixafor reduced total score in comparison to group 2M (2.80 ± 2.35 for Influenza + Plerixafor 20 mg/kg vs 4.50 ± 2.72 for Influenza). More specifically, this reduction was mainly due to the diminution of necrosis/cell death (0.40 ± 0.70 for Influenza + Plerixafor 20 mg/kg vs 0.90 ± 0.74 for Influenza) and edema, HM and congestion scores (0.60 ± 0.52 for Influenza + Plerixafor 20 mg/kg vs 1.50 ± 1.35 for Influenza) while it did not affect the inflammation score (1.60 ± 1.17 for Influenza + Plerixafor 20 mg/kg vs 1.90 ± 0.88 for Influenza).

**Table 1- Total score according Meyerholz and Beck**

| | **Mean total score** | **SD** |
|---|---|---|
| **1M - Non infected** | 0,00 *** | 0,00 |
| **2M - Influenza** | 4,50 | 2,72 |
| **3M - Influenza + Plerixafor 2,5 mg/kg** | 2,70 | 2,21 |
| **4M - Influenza + Plerixafor 5 mg/kg** | 4,00 | 1,63 |
| **5M - Influenza + Plerixafor 10 mg/kg** | 2,90 | 1,79 |
| **6M - Influenza + Plerixafor 20 mg/kg** | 2,80 | 2,35 |

**Table 2- Necrosis cell death score according Meyerholz and Beck**

| | **Mean necrosis/cell death score** | **SD** |
|---|---|---|
| **1M - Non infected** | 0,00 * | 0,00 |
| **2M - Influenza** | 0,90 | 0,74 |
| **3M - Influenza + Plerixafor 2,5 mg/kg** | 0,50 | 0,53 |
| **4M - Influenza + Plerixafor 5 mg/kg** | 0,80 | 0,42 |
| **5M - Influenza + Plerixafor 10 mg/kg** | 0,50 | 0,53 |
| **6M - Influenza + Plerixafor 20 mg/kg** | 0,40 | 0,70 |

**Table 3: Edema, hyaline membrane and congestion score according Meyerholz and Beck**

| | **Mean edema/HM/congestion score** | **SD** |
|---|---|---|
| **1M - Non infected** | 0,00 ** | 0,00 |
| **2M - Influenza** | 1,50 | 1,35 |
| **3M - Influenza + Plerixafor 2,5 mg/kg** | 0,70 | 0,82 |
| **4M - Influenza + Plerixafor 5 mg/kg** | 0,90 | 0,74 |
| **5M - Influenza + Plerixafor 10 mg/kg** | 0,50 | 0,53 |
| **6M - Influenza + Plerixafor 20 mg/kg** | 0,60 | 0,52 |

**Table 4- Inflammation score according Meyerholz and Beck**

| | **Mean inflammation score** | **SD** |
|---|---|---|
| **1M** - **Non infected** | 0,00 | 0,00 |
| **2M** - **Influenza** | 1,90 | 0,88 |
| **3M - Influenza + Plerixafor 2,5 mg/kg** | 1,40 | 0,97 |
| **4M - Influenza + Plerixafor 5 mg/kg** | 2,00 | 0,47 |
| **5M - Influenza + Plerixafor 10 mg/kg** | 1,70 | 0,97 |
| **6M - Influenza + Plerixafor 20 mg/kg** | 1,60 | 1,17 |

### Example 4: ELISA on lung homogenates

Lung were collected at termination on day 7 for 10 mice per group for groups 2M to 6M and 6 mice for group 1M. ELISA were performed on lung homogenates to evaluate the levels of IL-6, CRP, C5a, CXCL-1, IL-1β, IL-17 and TNFα.

### IL-6 levels in lung homogenates

The IL-6 pro-inflammatory cytokine concentration was evaluated in lung homogenates.

IL-6 concentration in lung homogenates of non-infected mice was detected at low concentration. The infection with the Influenza A virus significantly increased the IL-6 concentration (15055,10 ± 9438,68 pg/ml/g for Influenza group vs 2412,49 ± 639,38 pg/ml/g for non-infected group, p<0,05). Treatment of infected mice with Plerixafor at 2,5, 5 and 10 mg/kg did not significantly modulate the IL-6 levels in comparison to Influenza group. However, treatment with the higher dose of Plerixafor at 20 mg/kg significantly decreased this concentration (5408,82 ± 3539,57 pg/ml/g for Influenza + Plerixafor 20 mg/kg vs 15055,10 ± 9438,68 pg/ml/g for Influenza, p<0,05). Mean concentrations of IL-6 are presented in **Table 5** and **Figure 6****.**

**Table 5- Mean IL-6 levels in lung homogenates**

| | **Mean IL-6 levels (pg/ml/g of tissue)** | **SD** |
|---|---|---|
| **1M** - **Non infected** | 2412,49 * | 639,38 |
| **2M** - **Influenza** | 15055,10 | 9438,68 |
| **3M - Influenza + Plerixafor 2,5 mg/kg** | 11166,93 | 7299,11 |
| **4M - Influenza + Plerixafor 5 mg/kg** | 11059,46 | 7837,86 |
| **5M - Influenza + Plerixafor 10 mg/kg** | 13927,98 | 10933,08 |
| **6M - Influenza + Plerixafor 20 mg/kg** | 5408,82 * | 3539,57 |

### Example 5: Results: CRP levels in lung homogenates

CRP concentration, an acute phase protein which increase following IL-6 secretion was also evaluated in lung homogenates.

CRP concentration in lung homogenates of non-infected mice was detected at relatively high concentration. The infection with the Influenza A virus significantly increased the CRP concentration (9243070,24 ± 3683588,82 pg/ml/g for Influenza group vs 2718616,67 ± 824010,10 pg/ml/g for non-infected group, p<0,01). Treatment of infected mice with Plerixafor at 2,5, 5 and 10 mg/kg did not significantly modulate the CRP levels. However, treatment with the higher dose of Plerixafor at 20 mg/kg significantly decreased this concentration (4488307,08 ± 2059120,87 pg/ml/g for Influenza + Plerixafor 20 mg/kg vs 9243070,24 ± 3683588,82 pg/ml/g for Influenza, p<0,01). Mean concentrations of CRP are presented in **Table 6** and **Figure 7****.**

**Table 6- Mean CRP levels in lung homogenates**

| | **Mean CRP levels (pg/ml/g of tissue)** | **SD** |
|---|---|---|
| **1M** - **Non infected** | 2718616,67 ** | 824010,10 |
| **2M** - **Influenza** | 9243070,24 | 3683588,82 |
| **3M - Influenza + Plerixafor 2,5 mg/kg** | 9241738,89 | 4298459,06 |
| **4M - Influenza + Plerixafor 5 mg/kg** | 7792352,38 | 3681090,68 |
| **5M - Influenza + Plerixafor 10 mg/kg** | 8406400,43 | 4804030,06 |
| **6M - Influenza + Plerixafor 20 mg/kg** | 4488307,08 ** | 2059120,87 |

### Example 6: C5a levels in lung homogenates

C5a concentration, a potent chemokine which stimulates the locomotion of polymorphonuclear leukocytes and directs their migration toward sites of inflammation, was evaluated in lung homogenates.

C5a concentration in lung homogenates of non-infected mice was detected at relatively high concentration. The infection with the Influenza A virus significantly increased the C5a concentration (2540416,67 ± 529299,86 pg/ml/g for Influenza group vs 2540416,67 ± 529299,86 pg/ml/g for non-infected group, p<0,05). Treatment of infected mice with Plerixafor at 2,5, 5 and 10 mg/kg did not significantly modulate the C5a levels. However, treatment with the higher dose of Plerixafor at 20 mg/kg significantly decreased this concentration (2220172,76 ± 972810,94 pg/ml/g for Influenza + Plerixafor 20 mg/kg vs 3758066,67± 1249116,75 pg/ml/g for Influenza, p<0,01). Mean concentrations of C5a are presented in **Table 7** and **Figure 8****.**

**Table 7- Mean C5a levels in lung homogenates**

| | **Mean C5a levels (pg/ml/g of tissue)** | **SD** |
|---|---|---|
| **1M** - **Non infected** | 2540416,67 * | 529299,86 |
| **2M** - **Influenza** | 3758066,67 | 1249116,75 |
| **3M - Influenza + Plerixafor 2,5 mg/kg** | 3699919,44 | 1171406,26 |
| **4M - Influenza + Plerixafor 5 mg/kg** | 3440259,52 | 1027372,71 |
| **5M - Influenza + Plerixafor 10 mg/kg** | 3519038,68 | 1073136,22 |
| **6M - Influenza + Plerixafor 20 mg/kg** | 2220172,76 ** | 972810,94 |

### Example 7: Results: IL-1β levels in lung homogenates

The IL-1β pro-inflammatory cytokine concentration was evaluated in lung homogenates.

IL-1β concentration in lung homogenates of non-infected mice was detected at low concentration. The infection with the Influenza A virus significantly increased the IL-1β concentration (3502,64 ± 2441,68 pg/ml/g for Influenza group vs 1347,22 ± 184,31 pg/ml/g for non-infected group, p<0,05). Treatment of infected mice with Plerixafor at 2,5, 5, 10 and 20 mg/kg did not significantly modulate the IL-1β levels. Mean concentrations of IL-1β are presented in **Table 8** and **Figure 9****.**

**Table 8- Mean IL-1β levels in lung homogenates**

| | **Mean IL-1β levels (pg/ml/g of tissue)** | **SD** |
|---|---|---|
| **1M** - **Non infected** | 1347,22 * | 184,31 |
| **2M - Influenza** | 3502,64 | 2441,68 |
| **3M - Influenza + Plerixafor 2,5 mg/kg** | 4317,27 | 2003,62 |
| **4M - Influenza + Plerixafor 5 mg/kg** | 2534,11 | 1071,45 |
| **5M - Influenza + Plerixafor 10 mg/kg** | 3319,66 | 2901,44 |
| **6M - Influenza + Plerixafor 20 mg/kg** | 2805,78 | 1469,82 |

### Example 8: IL-17 levels in lung homogenates

The IL-17 pro-inflammatory cytokine concentration was evaluated in lung homogenates.

IL-17 concentration in lung homogenates of non-infected mice was detected at low concentration. The infection with the Influenza A virus significantly decreased the IL-17 concentration (575,58 ± 138,84 pg/ml/g for Influenza group vs 874,47 ± 209,36 pg/ml/g for non-infected group, p<0,01). Treatment of infected mice with Plerixafor at 2,5, 5, 10 and 20 mg/kg did not significantly modulate the IL-17 levels. Mean concentrations of IL-17 are presented in **Table 9** and **Figure 10****.**

**Table 9- Mean IL-17 levels in lung homogenates**

| | **Mean IL-17 levels (pg/ml/g of tissue)** | **SD** |
|---|---|---|
| **1M - Non infected** | 874,47 ** | 209,36 |
| **2M - Influenza** | 575,58 | 138,84 |
| **3M - Influenza + Plerixafor 2,5 mg/kg** | 748,08 | 208,61 |
| **4M - Influenza + Plerixafor 5 mg/kg** | 663,57 | 292,47 |
| **5M - Influenza + Plerixafor 10 mg/kg** | 799,98 | 565,05 |
| **6M - Influenza + Plerixafor 20 mg/kg** | 603,22 | 246,95 |

### Example 9: TNFα levels in lung homogenates

The TNFα concentration, an important mediator of inflammatory and immune functions, was evaluated in lung homogenates.

TNFα concentration in lung homogenates of non-infected mice was detected at low concentration. The infection with the Influenza A virus did not modulate the TNFα concentration (11038 ± 6765,77 pg/ml/g for Influenza group vs 11879,58 ± 1803,85 pg/ml/g for non-infected group). Treatment of infected mice with Plerixafor at 2,5, 5 and 10 mg/kg did not significantly modulate the TNFα levels. However, treatment with the higher dose of Plerixafor at 20 mg/kg slightly decreased this concentration (6224,51 ± 3014,54 pg/ml/g for Influenza + Plerixafor 20 mg/kg vs 11038,49 ± 6765,77 pg/ml/g for Influenza). Mean concentrations of TNFα are presented in **Table 10** and **Figure 11****.**

**Table 10- Mean TNFα levels in lung homogenates**

| | **Mean TNFα levels (pg/ml/g of tissue)** | **SD** |
|---|---|---|
| **1M - Non infected** | 11879,58 | 1803,85 |
| **2M - Influenza** | 11038,49 | 6765,77 |
| **3M - Influenza + Plerixafor 2,5 mg/kg** | 8983,01 | 3672,52 |
| **4M - Influenza + Plerixafor 5 mg/kg** | 9307,38 | 4103,92 |
| **5M - Influenza + Plerixafor 10 mg/kg** | 11138,01 | 6478,98 |
| **6M - Influenza + Plerixafor 20 mg/kg** | 6224,51 | 3014,54 |

## Claims

1. A compound of formula (I) or a pharmaceutically acceptable salt and/or hydrate thereof, for use in the treatment of a viral pulmonary disease in a subject, said disease being different from COVID-19,
wherein formula (I) is:
Z-R-A-R'-Y (I),
wherein:
- Z and Y, which are identical or different, represent:
∘ an alkyl group having from 1 to 6 carbon atoms, optionally substituted by a hydroxyl group, a halogen atom, a CN group, a CF3 group, a SO group, a NO2 group, an amine group, a difluor or an alkyl, S-alkyl or O-alkyl group having from 1 to 6 carbon atoms, or
∘ an aryl or heteroaryl group having from 3 to 6 carbon atoms, optionally substituted by a hydroxyl group, a halogen atom, a CN group, a CF3 group, a SO group, a NO2 group, an amine group, a difluor or an alkyl, S-alkyl or O-alkyl group having from 1 to 6 carbon atoms, or
∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, or
∘ a D₁D₂N- group, wherein D₁ and D₂, which may be identical or different, represent:
▪ a hydrogen atom, or
▪ an alkyl group having from 1 to 6 carbon atoms optionally substituted by at least one hydroxyl group, a halogen atom, a CF3 group, a CN group, an amine group, or an alkyl, O-alkyl or S-alkyl group having from 1 to 12 carbon atoms, or
▪ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF3 group, CN group, amine group, or alkyl, O-alkyl or S-alkyl group having from 1 to 12 carbon atoms, or
▪ D₁ and D₂ are linked together to from a N-containing aryl or heteroaryl group having from 3 to 12 carbon atoms and optionally substituted by at least one amine group optionally substituted by an alkylheteroaryl group having from 3 to 12 carbon atoms, and
- A represents:
∘ an aryl or heteroaryl group having from 3 to 12 carbon atoms, or
∘ an alkyl group having from 1 to 6 carbon atoms, or
∘ -R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF3 group, a CN group or an alkyl, O-alkyl or S-alkyl group having from 1 to 6 carbon atoms optionally substituted by a hydroxyl group, an amine group or an O-alkyl group having from 1 to 6 carbon atoms, and
- R and R', which are identical or different, represent an alkyl group having from 1 to 6 carbon atoms or a R₁NR₂R₃ group or a single bond, and
- R₁ represents a single bond or an alkyl group having from 1 to 6 carbon atoms, and
- R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms.

2. The compound for use according to claim **1,** wherein:
- Z and Y, which are identical or different, represent:
∘ an alkyl group having from 1 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
∘ an aryl or heteroaryl group having from 3 to 6 carbon atoms, optionally substituted by a hydroxyl group, a halogen atom, a CN group, a CF₃ group, a SO group, a NO₂ group, an amine group, a difluor or an alkyl, S-alkyl or O-alkyl group having from 1 to 6 carbon atoms, or
∘ a cyclic or heterocyclic polyamine group having from 9 to 32 ring members and from 3 to 8 amine groups in the ring spaced by 2 or more carbon atoms from each other, optionally comprising an aryl or heteroaryl group having from 3 to 6 carbon atoms, a heteroatom, and optionally substituted by a halogen atom, a hydroxyl group, a heteroatom, an alkyl, S-alkyl or O-alkyl group having from 1 to 6 carbon atoms or an aryl or heteroaryl group having from 3 to 6 carbon atoms, and
- A represents an aryl or heteroaryl group having from 3 to 12 carbon atoms, and
- R and R', which are identical or different, represent an alkyl group having from 1 to 6 carbon atoms or a R₁NR₂R₃ group, and
- R₁ represents a single bond or an alkyl group having from 1 to 6 carbon atoms, and
- R₂ and R₃, which are identical or different, represent a hydrogen atom, an amine group, an alkyl group having from 1 to 6 carbon atoms or an aryl or heteroaryl group having from 3 to 6 carbon atoms, wherein said aryl or heteroaryl group having from 3 to 6 carbon atoms is optionally substituted by an alkyl group having from 1 to 6 carbon atoms.

3. The compound for use according to claim **1,** wherein said compound of formula (I) is represented by the formula (II): 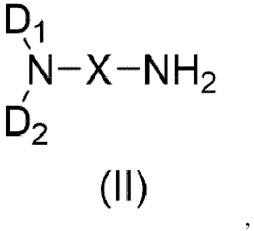 wherein:
- D₁ and D₂, which may be identical or different, represent:
∘ an alkyl group having from 1 to 6 carbon atoms optionally substituted by at least one hydroxyl group, a halogen atom, a CF₃ group, a CN group, an amine group, or an alkyl, O-alkyl or S-alkyl group having from 1 to 12 carbon atoms, or
∘ an aryl, an heteroaryl, a cycloalkyl, a heterocycloalkyl, an alkylaryl, an alkylheteroaryl or an alkylheteropolyaryl group having from 3 to 12 carbon atoms, optionally substituted by at least one hydroxyl group, halogen atom, CF3 group, CN group, amine group, or alkyl, O-alkyl or S-alkyl group having from 1 to 12 carbon atoms, or
∘ D₁ and D₂ are linked together to from a N-containing aryl or heteroaryl group having from 3 to 12 carbon atoms and optionally substituted by at least one amine group optionally substituted by an alkylheteroaryl group having from 3 to 12 carbon atoms, and
- X represents:
∘ an alkyl group having from 1 to 6 carbon atoms, or
∘ -R₄-Y'-R₅-, wherein R₄ and R₅ which are identical or different represent an alkyl group having from 1 to 6 carbon atoms and Y' represents an aryl or heteroaryl group having from 3 to 6 carbon atoms, optionally substituted by a halogen atom, a hydroxyl group, an amide group, an amine group, an alkoxy group, an ester group, a CF3 group, a CN group or an alkyl, O-alkyl or S-alkyl group having from 1 to 6 carbon atoms optionally substituted by a hydroxyl group, an amine group or an O-alkyl group having from 1 to 6 carbon atoms.

4. The compound for use according to claim **1** or **2,** wherein said compound is 1,1'-[1,4-phenylene-bis(methylene)]-bis-1,4,8,11-tetraazacyclotetradecane.

5. The compound for use according to any one of claim **1** to **4** wherein the viral pulmonary disease leads to an acute and/or chronic respiratory distress syndrome.

6. The compound for use according to any one of claim **1** to **5** wherein the viral pulmonary disease is caused by a virus chosen among influenza virus, respiratory syncytial virus, parainfluenza viruses, metapneumovirus, rhinovirus, coronaviruses such as severe acute respiratory syndrome coronavirus (SARS-CoV) coronavirus except SARS-CoV2 or Middle East respiratory syndrome coronavirus (MERS-CoV), adenoviruses, and bocaviruses.

7. The compound for use according to any one of claim **1** to **6** wherein the viral pulmonary disease is chosen among influenza, bronchiolitis, laryngotracheitis, pneumopathy, pneumonia.

8. A pharmaceutical composition for use in treating a viral pulmonary disease said disease being different from COVID-19 comprising the compound of any one of claims **1** to **7** and a pharmaceutically acceptable excipient.

9. The pharmaceutical composition for use according to claim **8** further comprising at least one other active agent.

10. The pharmaceutical composition for use according to claim **9** wherein the active agent is an anti-inflammatory selected from the group comprising nonsteroidal anti-inflammatory drugs (NSAIDs) such as Ibuprofen, Naproxen, Celecoxib, Diclofenac, Indomethacin, Oxaprozin, and Piroxicam or steroidal anti-inflammatory drugs such as Cortisone, Dexamethasone, Hydrocortisone, Methylprednisolone or Prednisolone.

11. The pharmaceutical composition for use according to claim **9** or **10** wherein the active agent is selected from the group comprising an immune checkpoint inhibitor (ICI), such as an inhibitor of PD-1, an inhibitor of PD-L1, an inhibitor of CTLA-4 and an inhibitor of LAG3, preferably, the active agent is an inhibitor of PD-1 and an inhibitor of PD-L1.

12. The pharmaceutical composition for use according to any one of claims **8** to **11** wherein the pharmaceutical composition is administrated in a therapeutically effective amount.

13. The pharmaceutical composition for use according to any one of claims **8** to **12** wherein the pharmaceutical composition is administrated via parenteral route comprising intravenous, intramuscular, subcutaneous or intradermal route.

14. The pharmaceutical composition for use according to any one of claims **8** to **13** wherein the compound is Plerixafor and wherein Plerixafor is administrated via subcutaneous route at a dosage from 0,24 mg/kg/day to 1,44 mg/kg/day during at least 7 days.

15. The pharmaceutical composition for use according to any one of claims **8** to **13** wherein the compound is Plerixafor and wherein plerixafor is administrated intravenously at a dosage from 10 µg/kg/hour to 80 µg/kg/hour continuously during at least 7 days.
